# EUROPEAN PATENT SPECIFICATION

(11) **EP 4 076 229 B1**
(45) Date of publication and mention of the grant of the patent: **23.07.2025**
(21) Application number: 20835691.5
(22) Date of filing: 10.12.2020
(51) Int. Cl.: A61B 17/3207, A61B 17/00

(54) **ATHERECTOMY DEVICES INCLUDING SEALED DRIVE SHAFTS**
ATHEREKTOMIEVORRICHTUNGEN MIT ABGEDICHTETEN ANTRIEBSWELLEN
DISPOSITIFS D'ATHÉRECTOMIE COMPRENANT DES ARBRES D'ENTRAÎNEMENT SCELLÉS

(30) Priority: 20.12.2019 US 201962951000 P
(43) Date of publication of application: 26.10.2022
(73) Proprietor: Koninklijke Philips N.V., 5656 AG Eindhoven (NL); Philips Image Guided Therapy Corporation, San Diego CA 92130 (US)
(72) Inventor: POMBO, August Christopher, 5656 AE Eindhoven (NL); ESCUDERO, Paul Q, 5656 AE Eindhoven (NL); ROWE, Douglas, 5656 AE Eindhoven (NL)
(74) Representative: Philips Intellectual Property & Standards
(86) International application number: PCT/EP2020/085404
(87) International publication number: WO 2021/122253

(56) References cited:
- EP-A1- 3 222 228
- WO-A2-2015/017114
- US-A1- 2008 097 499

## Description

### FIELD OF THE DISCLOSURE

The devices and methods described herein generally relate to treatment of occluded body lumens, such as the removal of occlusive material from a blood vessel or other body parts.

### BACKGROUND

Peripheral and interventional cardiology is a medical specialty that relates to treatment of various forms of cardiovascular disease, including coronary artery disease and peripheral vascular disease. Coronary artery disease and peripheral vascular disease can arise due to the narrowing of the arteries by atherosclerosis (also called arteriosclerosis). Coronary artery disease generally affects arteries of the heart-arteries that carry blood to cardiac muscles and surrounding tissue. Peripheral vascular disease refers to various diseases of the vascular system outside the heart and brain, which carries blood, for example, to the legs.

Atherosclerosis commonly affects the medium and large arteries, and may occur when fat, cholesterol, and other substances build up on the walls of arteries and form fleshy or hard/calcified structures called plaques/lesions. As plaque forms within an arterial wall, the artery may narrow and become less flexible, which may make it more difficult for blood to flow therethrough. In the peripheral arteries, the plaque is typically not localized, but can extend in length along the axis of the artery for as much as 10 mm or more (in some instance up to 400 mm or more).

Pieces of plaque can break off and move through the affected artery to smaller blood vessels, which may in some instances block them and may result in tissue damage or tissue death (embolization). In some cases, the atherosclerotic plaque may be associated with a weakening of the wall of the affected artery, which can lead to an aneurysm. Minimally invasive surgeries may be performed to remove plaque from arteries in an effort to alleviate or help prevent the complications of atherosclerosis.

A number of interventional surgical methodologies may be used to treat atherosclerosis. In balloon angioplasty, for example, a physician may advance a collapsed, intravascular balloon catheter into a narrowed artery, and may inflate the balloon to macerate and/or displace plaque against the vessel wall. A successful angioplasty may help reopen the artery and allow for improved blood flow. Often, balloon angioplasty is performed in conjunction with the placement of a stent or scaffold structure within the artery to help minimize re-narrowing of the artery. Balloon angioplasty, however, can stretch the artery and induce scar tissue formation, while the placement of a stent can cut arterial tissue and also induce scar tissue formation. Scar tissue formation may lead to restenosis of the artery. In some instances, balloon angioplasty can also rip the vessel wall.

Atherectomy is another treatment methodology for atherosclerosis, and involves the use of an intravascular device to mechanically remove (that is, debulk) plaque from the wall of the artery. Atherectomy devices may allow for the removal of plaque from the wall of an artery, reducing the risk of stretching, cutter, or dissecting the arterial wall and causing tissue damage that leads to restenosis. In some instances, atherectomy may be used to treat restenosis by removing scar tissue.

Unfortunately, some atherectomy devices suffer from structural and performance limitations. For example, rotating cutting elements or assemblies of some atherectomy may be driven by coiled drive shafts. When such shafts are rotated in a curve of the vasculature, the coils open slightly and permit plaque and fluids to penetrate the shaft and enter an internal guide wire lumen. The plaque and fluids in the guide wire lumen increase friction between the shaft and a guide wire within the lumen, which can adversely impact device performance. Accordingly, it is desirable to provide improved atherectomy devices and methods.

US 2008/097499 A1, according to its abstract, relates to a device suitable for removing material from a living being, featuring at least an aspiration pump, powered by a motor. The aspiration pump and any optional infusate pump preferably feature a helical pumping mechanism, and operate at a high rate of rotation, thereby ensuring adequate pumping performance and flexibility. The helical pumping mechanism may be a helical coiled wire about a central core tube. The helical coil wire, whether together with, or independent of, the core tube, may be rotated to cause a pumping action. Additionally, a narrow crossing profile is maintained, ensuring that the device may reach more tortuous regions of the vasculature. In one embodiment, the system comprises a wire-guided mono-rail catheter with a working head mounted on a flexible portion of the catheter that can laterally displace away from the guide wire to facilitate thrombus removal. The working head may be operated to separate and move away from the guide wire to come within a closer proximity of the obstructive material to more effectively remove it from the vessel.

WO 2015/017114 A1, according to its abstract, relates to devices and components for use in performing an atherectomy. The atherectomy devices may have a handle, a cutter assembly, and a catheter or catheter assembly therebetween. The cutter assembly may include a housing and a cutter comprising a proximal cutter and a distal cutter, each of which may be rotated relative to the atherectomy device to cut occlusive material.

EP 3222228 A1, according to its abstract, relates to an atherectomy device for removing deposits such as plaque from an interior of a vessel including an outer member, an inner member and a rotatable shaft positioned for rotational movement within the inner member. The outer and inner members are fixed axially. A rotatable tip is mounted to the distal region of the rotatable shaft for rotation about its longitudinal axis upon rotation of the shaft to remove deposits from the vessel.

### SUMMARY

The invention provides an atherectomy device according to claim 1. Further embodiments of the invention are provided in the dependent claims. The present disclosure presents an atherectomy device for removing occlusive material from the vasculature of a subject. The atherectomy device includes a distal cutter assembly, and the cutter assembly includes a housing and a cutting element carried by the housing. The cutting element includes at least one cutting blade. The cutting element is rotatable relative to the housing to cause the at least one cutting blade to cut the occlusive material and move the occlusive material into the housing. The device further includes a catheter coupled to the distal cutter assembly. The catheter includes a sheath and a drive shaft disposed within the sheath and coupled to the cutting element. The drive shaft is rotatable relative to the sheath to rotate the cutting element relative to the housing. The drive shaft and the sheath define therebetween a material removal passageway for receiving the occlusive material from the housing. The drive shaft includes a coil having an inner lumen. A jacket is disposed outwardly of the coil, and the jacket inhibits the occlusive material in the material removal passageway from passing through the coil and entering the inner lumen.

According to the present disclosure, the jacket comprises a polymer.

According to the present disclosure, the polymer is a hydrophobic polymer.

According to the present disclosure, the hydrophobic polymer is one of *polytetrafluoroethylene* (PTFE) and fluorinate ethylene propylene (FEP).

According to the present disclosure, the coil is an inner coil, and further comprising an outer coil disposed outwardly from the inner coil, the outer coil being configured to proximally move the occlusive material in the material removal passageway upon rotation of the drive shaft relative to the sheath, and wherein the jacket is disposed between the inner coil and the outer coil.

According to the present disclosure, the atherectomy device further comprises an intermediate coil disposed between the inner coil and the jacket.

The present disclosure presents an atherectomy device for removing occlusive material from the vasculature of a subject. The atherectomy device includes a distal cutter assembly including a housing and a cutting element carried by the housing. The cutting element includes at least one cutting blade, and the cutting element is rotatable relative to the housing to cause the at least one cutting blade to cut the occlusive material and move the occlusive material into the housing. A catheter is coupled to the distal cutter assembly. The catheter includes a sheath that is configured to receive the occlusive material from the housing. The catheter further includes a drive shaft disposed within the sheath and coupled to the cutting element. The drive shaft is rotatable relative to the sheath to rotate the cutting element relative to the housing. The drive shaft includes an inner coil having an inner lumen and an outer coil disposed outwardly from the inner coil. The outer coil is configured to proximally move the occlusive material in a material removal passageway disposed between the drive shaft and the sheath upon rotation of the drive shaft relative to the sheath. A sealing layer is disposed between the inner coil and the outer coil. The sealing layer inhibits the occlusive material in the material removal passageway from passing through the inner coil and entering the inner lumen.

According to the present disclosure, the sealing layer comprises a polymer.

According to the present disclosure, the polymer is a hydrophobic polymer.

According to the present disclosure, the hydrophobic polymer is one of *polytetrafluoroethylene* (PTFE) and fluorinate ethylene propylene (FEP).

According to the present disclosure, the atherectomy device further comprises an intermediate coil disposed between the inner coil and the sealing layer.

The present invention presents an atherectomy device for removing occlusive material from the vasculature of a subject. The atherectomy device includes a distal cutter assembly including a housing and a cutting element carried by the housing. The cutting element has at least one cutting blade. The cutting element is rotatable relative to the housing to cause the at least one cutting blade to cut the occlusive material and move the occlusive material into the housing. The atherectomy device further includes a catheter coupled to the distal cutter assembly. The catheter includes a sheath that is configured to receive the occlusive material from the housing. A drive shaft is disposed within the sheath and coupled to the cutting element. The drive shaft is rotatable relative to the sheath to rotate the cutting element relative to the housing. The drive shaft includes an inner coil having an inner lumen. An outer conveyor is disposed outwardly from the inner coil. The outer conveyor is configured to proximally move the occlusive material in a material removal passageway disposed between the drive shaft and the sheath upon rotation of the drive shaft relative to the sheath. A sealing layer is disposed between the inner coil and the outer conveyor. The sealing layer inhibits the occlusive material in the material removal passageway from passing through the inner coil and entering the inner lumen.

In an embodiment of the present invention, the sealing layer comprises a polymer.

In an embodiment of the present invention, the polymer is a hydrophobic polymer.

In an embodiment of the present invention, the hydrophobic polymer is one of *polytetrafluoroethylene* (PTFE) and fluorinate ethylene propylene (FEP).

In an embodiment of the present invention, the atherectomy device further comprises an intermediate coil disposed between the inner coil and the sealing layer.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, V₁ and Zₒ).

The term "a" or "an" entity refers to one or more of that entity. As such, the terms "a" (or "an"), "one or more" and "at least one" may be used interchangeably herein. It is also to be noted that the terms "comprising", "including", and "having" may be used interchangeably.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure may be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 is a side view of an atherectomy system according to an embodiment of the present disclosure.
FIG. 2A is a detail side view of a distal portion of the atherectomy system of FIG. 1.
FIG. 2B is a detail perspective view of the distal portion of the atherectomy system of FIG. 1.
FIG. 2C is a detail transverse sectional view of the distal portion of the atherectomy system of FIG. 2A.
FIG. 3A is a perspective view of a distal cutting element of the atherectomy system of FIG. 1.
FIG. 3B is a side view of the distal cutting element of FIG. 3A.
FIG. 4A is a perspective view of a proximal cutting element of the atherectomy system of FIG. 1.
FIG. 4B is a front view of the proximal cutting element of FIG. 4A.
FIG. 5 is a transverse sectional view of a drive shaft of the atherectomy system of FIG. 1.

It should be understood that the drawings are not necessarily to scale. In certain instances, details that are not necessary for an understanding of the disclosure or that render other details difficult to perceive may have been omitted. It should be understood, of course, that the disclosure is not necessarily limited to the particular embodiments illustrated herein.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter as well as additional items.

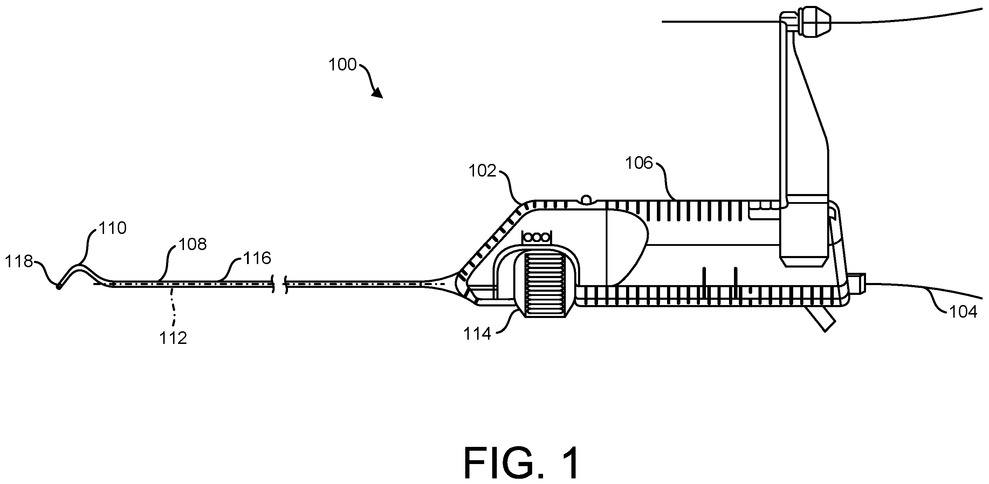

The present disclosure relates generally to devices, systems, and methods for mechanical atherectomy. Referring to FIG. 1, there is shown an exemplary embodiment of the atherectomy systems described herein. The atherectomy system 100 includes an intravascular atherectomy device 102 and a guide wire 104 over which the atherectomy device 102 may be deployed. In some embodiments, the guide wire 104 is silicon-coated or non-coated (bare), or otherwise free of a *polytetrafluoroethylene* (PTFE) coating. Atherectomy systems according to some embodiments of the present disclosure comprise a guide wire 104 that includes a PTFE coating, or atherectomy systems according to some embodiments of the present disclosure lack a guide wire 104.

With continued reference to FIG. 1, the atherectomy device 102 generally includes a handle 106 and a catheter 108. The handle 106 is configured to be grasped and manipulated by a user (for example, a medical professional) during an atherectomy procedure. The catheter 108 is coupled to and extends distally relative to the handle 106. The catheter 108 is configured to be positioned in the vasculature of a subject (for example, a patient) during an atherectomy procedure to facilitate removal of occlusive material (for example, plaque) therefrom. In some embodiments and as illustrated, a distal portion 110 of the catheter 108 has a curved shape or configuration. In some embodiments, the distal portion 110 of the catheter 108 normally has a curved configuration ("normally" being understood as the catheter 108 not being subjected to any external contact forces due to, for example, contact with blood vessel walls) and may be deflected to other configurations. In other embodiments, the distal portion 110 of the catheter 108 normally has a straight shape or configuration and may be deflected to other configurations. In some embodiments, the catheter 108 is selectively rotatable about a catheter rotational axis 112 relative to the handle 106 to facilitate appropriately positioning and or "sweeping" the distal portion 110 of the catheter 108 during an atherectomy procedure. In some embodiments and as illustrated, the handle 106 carries a rotatable knob or dial 114 for selectively rotating the catheter 108 relative to the handle 106. The catheter 108 includes an outer sheath 116, and the outer sheath 116 couples to a cutter assembly 118 that extends distally therefrom. The cutter assembly 118 is described in further detail below.

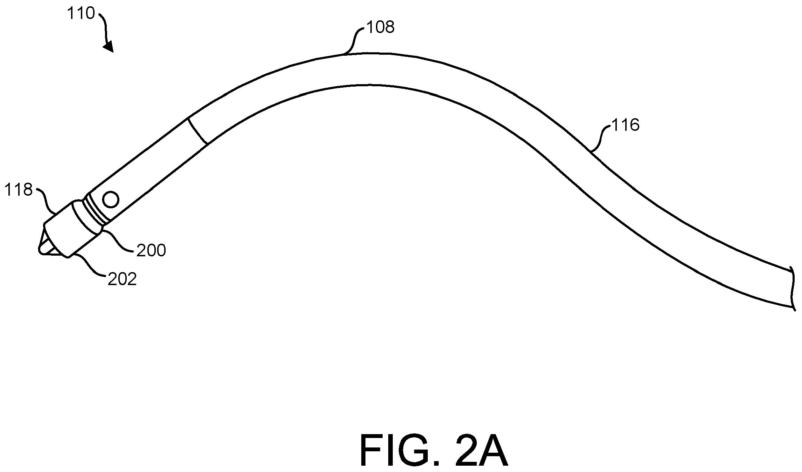

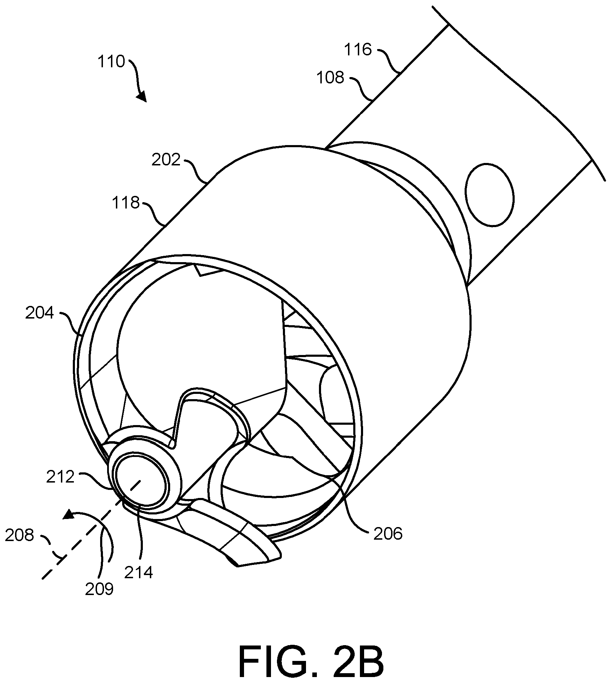

FIGS. 2A-2C illustrate the distal portion 110 of the catheter 108, including, among other components, the outer sheath 116 and the cutter assembly 118. The cutter assembly 118 includes a ferrule 200 that couples to the outer sheath 116 and extends distally therefrom. The cutter assembly 118 further includes a housing 202 that couples to the ferrule 200 and extends distally therefrom. The housing 202 rotatably carries cutting elements. Referring specifically to FIGS. 2B-2C, the housing 202 rotatably carries a first, or distal, cutting element 204 and a second, or proximal, cutting element 206. Rotation of the first cutting element 204 and the second cutting element 206 about a rotation axis 208 in a rotational direction 209 relative to the housing 202 causes the cutting elements 204, 206 to cut occlusive material and convey the occlusive material into the housing 202 (a process also referred to as "debulking").

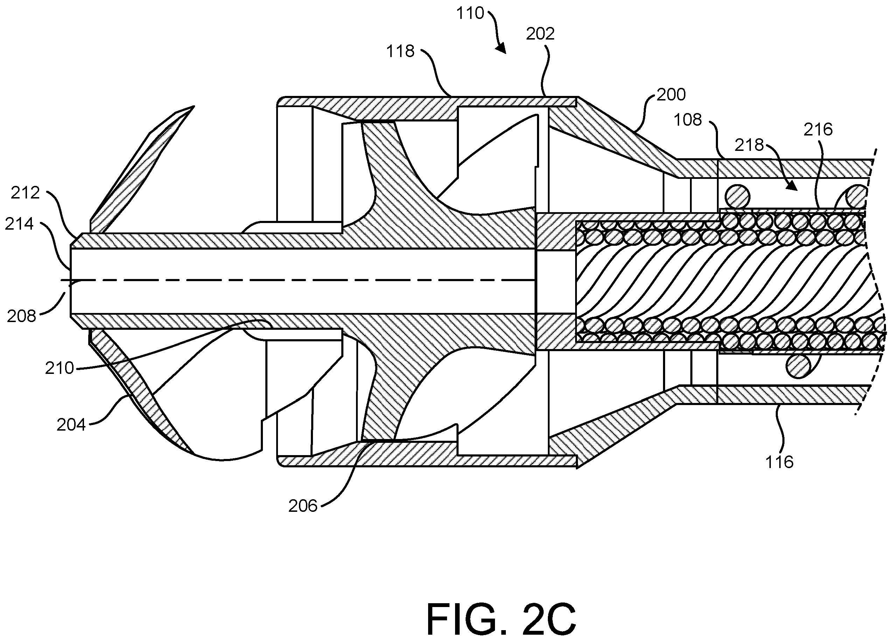

Still referring to FIGS. 2B-2C, the first cutting element 204 generally extends distally from the second cutting element 206 and the housing 202. The first cutting element 204 includes a central opening 210 (see FIG. 2C) for coupling to the second cutting element 206. The second cutting element 206 is generally disposed within the housing 202 and, in some embodiments and as illustrated, may be completely disposed within the housing 202. The second cutting element 206 is also generally disposed proximally from the first cutting element 204, although the second cutting element 206 includes a shaft or stem 212 that is received in the central opening 210. The stem 212 may couple to the first cutting element 204 in various manners. For example, the stem 212 may couple to the first cutting element 204 via welding. In some embodiments and as illustrated, the stem 212 extends distally relative to the first cutting element 204. The stem 212 includes an inner lumen 214 for receiving a guide wire 104 (shown elsewhere).

Referring specifically to FIG. 2C, the atherectomy device 102 further includes a rotatable drive shaft 216 that couples the first cutting element 204 and the second cutting element 206 to a prime mover (for example, a motor carried by the handle 106 - not shown). That is, the prime mover rotates the drive shaft 216, which in turn rotates the first cutting element 204 and the second cutting element 206 to facilitate cutting occlusive material and conveying the occlusive material into the housing 202. In some embodiments, the cutter assembly 118 captures the cut occlusive material from the blood without the use of vacuum aspiration. In other embodiments, vacuum aspiration may assist capture of the cut occlusive material. In either case, the housing 202 delivers the cut occlusive material to a material removal passageway 218 formed between the outer sheath 116 and the drive shaft 216. The occlusive material moves proximally through the material removal passageway 218 due to features of the drive shaft 216, as described in further detail below, and/or vacuum aspiration, and the occlusive material is thereby removed from the vasculature of the subject.

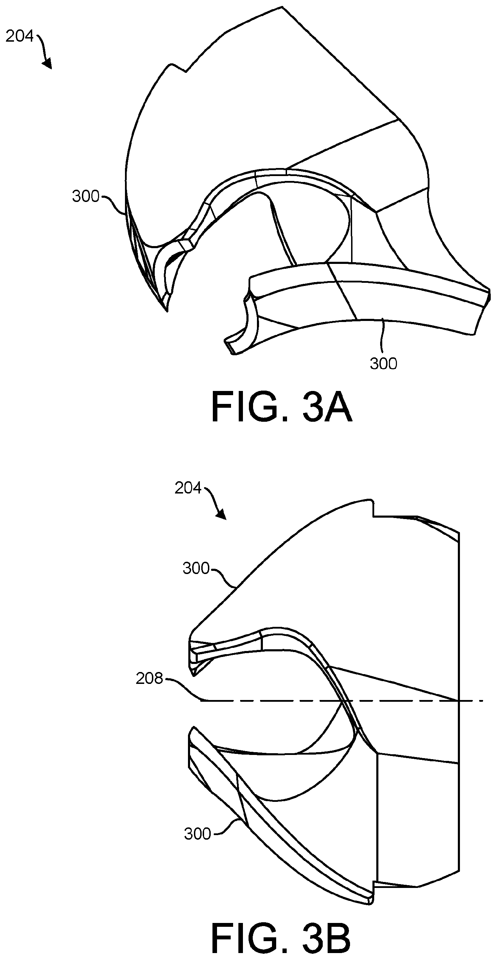

Referring to FIGS. 3A-3B, the first cutting element 204 includes one or more first, or distal, cutting flutes or blades 300 that extend distally relative to the housing 202 (shown elsewhere). In some embodiments and as illustrated, the first cutting element 204 includes two cutting blades 300. In some embodiments and as illustrated, one or more of the first cutting blades 300 extend helically relative to the rotation axis 208.

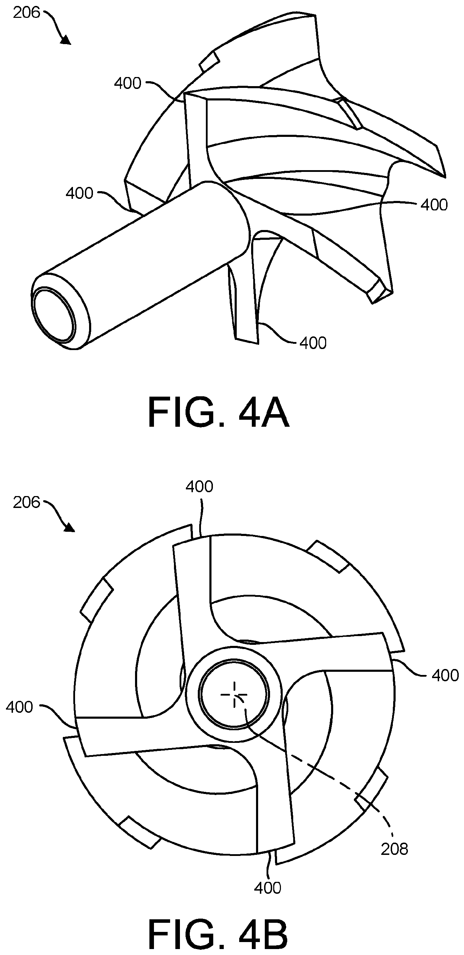

Referring now to FIGS. 4A-4B, the second cutting element 206 includes one or more second, or proximal, cutting flutes or blades 400. In some embodiments, the second cutting element 206 has two times the number of blades 400 as the first cutting element 204 (shown elsewhere). In some embodiments and as illustrated, the second cutting element 206 includes four cutting blades 400. In some embodiments and as illustrated, one or more of the second cutting blades 400 extend helically relative to the rotation axis 208.

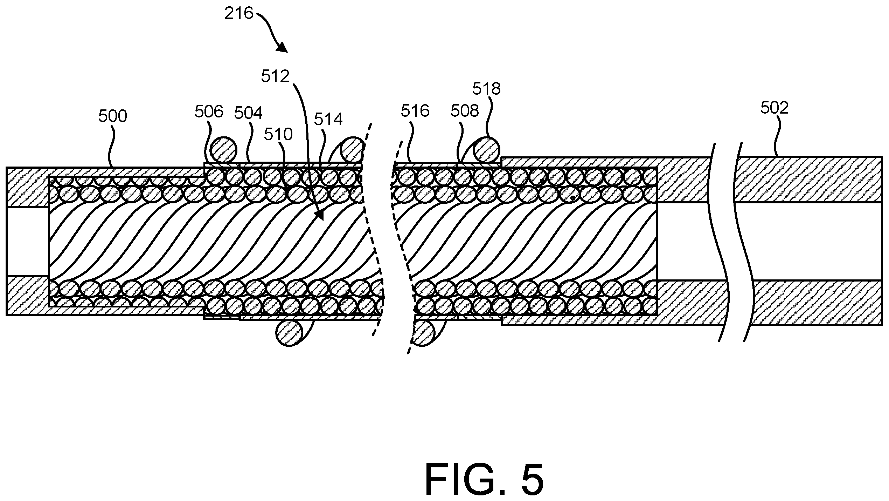

FIG. 5 illustrates a transverse sectional view the drive shaft 216. The drive shaft 216 generally includes a distal end coupling 500 that couples to the cutter assembly 118 (shown elsewhere), a proximal end coupling 502 that couples to the prime mover (not shown), and a coiled section 504 that extends between and couples the distal end coupling 500 to the proximal end coupling 502. The distal end coupling 500 is a generally cylindrical component that receives a portion of the coiled section 504. The distal end coupling 500 may be welded to and couple to the coiled section 504 via a distal solder joint 506. The distal end coupling 500 may be formed of a metal, such as stainless steel, and more specifically 304 stainless steel. Similarly, the proximal end coupling 502 is a generally cylindrical component that receives a portion of the coiled section 504. The proximal end coupling 502 may be welded to and couple to the coiled section 504 via a proximal solder joint 508. The proximal end coupling 502 may be formed of a metal, such as stainless steel, and more specifically 304 stainless steel.

With continued reference to FIG. 5, the coiled section 504 includes several components that provide flexibility to the drive shaft 216 and inhibit penetration by occlusive material. More specifically, the coiled section 504 includes an inner coil 510 that defines, together with the distal end coupling 500 and the proximal end coupling 502, an inner lumen 512 configured to receive the guide wire 104 (shown elsewhere). The inner coil 510 may have about 10 filars, and each filar may have a diameter of about 0.1016 mm (0.004 inches). The inner coil 510 may be right-hand wound. The inner coil 510 may be formed of a metal, such as stainless steel, and more specifically 304 stainless steel. The coiled section 504 also includes an intermediate coil 514 disposed radially outwardly from the inner coil 510. The intermediate coil 514 may have about 10 filars, and each filar may have a diameter of about 0.1016 mm (0.004 inches). The intermediate coil 514 may be lefthand wound. The intermediate coil 514 may be formed of a metal, such as stainless steel, and more specifically 304 stainless steel.

The coiled section 504 further includes a jacket 516, also referred to as an intermediate jacket and a sealing layer, disposed radially outwardly from the intermediate coil 514. The jacket 516 may extend over the majority of the length of the coiled section 504, more specifically, from the distal solder joint 506 to the proximal solder joint 508. The jacket 516 provides a seal for the intermediate coil 514 and the inner coil 510 or, stated another way, the jacket 516 inhibits the occlusive material in the material removal passageway 218 (shown elsewhere) from passing through the intermediate coil 514, through the inner coil 510, and entering the inner lumen 512. The jacket 516 may be formed of, for example, one or more polymers, more specifically a hydrophobic polymer, such as PTFE or fluorinate ethylene propylene (FEP). The jacket 516 may be formed over the intermediate coil 514, for example, in a heat-shrinking process, an extrusion process, or the like.

In some embodiments, the jacket 516 provides one or more advantages. For example, by inhibiting occlusive material from penetrating the drive shaft 216, the jacket 516 causes rotational friction between the drive shaft 216 and the guide wire 104 to remain relatively low and/or constant during use of the atherectomy device 102. In some embodiments, an atherectomy device 102 including the jacket 516 may be subjected to rotational friction between the drive shaft 216 and the guide wire 104 during use of the atherectomy device 102 that is reduced by about 96 percent compared to a similar atherectomy device lacking the jacket 516. As another example, the jacket 516 provides damping and reduces vibration of the drive shaft 216. As yet another example, the jacket 516 permits the inner lumen 512 of the drive shaft 216 to be used as a delivery lumen for providing, for example, a fluid to the subject, such as a therapeutic agent, nitroglycerin, saline solution, contrast solution, or the like.

Still referring to FIG. 5, the drive shaft 216 further includes an outer coil 518, also referred to as an outer conveyor or a screw pump, disposed radially outwardly from the jacket 516. The outer coil 518 conveys occlusive material proximally in the material removal passageway 218 as the drive shaft 216 rotates relative to the sheath. The outer coil 518 may have one filar, and the filar may have a diameter of about 0.1524 mm, that is 0.1524 mm ± 0.0254 mm (0.006 inches, that is 0.006 inches ± 0.001 inches). The outer coil 518 may have a pitch of about 0.762 mm (0.030 inches). The outer coil 518 may be right-hand wound. The outer coil 518 may be formed of a metal, such as stainless steel, and more specifically 304 stainless steel.

The foregoing discussion has been presented for purposes of illustration and description. The foregoing is not intended to limit the disclosure to the form or forms disclosed herein. In the foregoing Summary for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. The scope of the invention is defined by the appended claims.

## Claims

1. An atherectomy device (102) for removing occlusive material from a subject, the atherectomy device comprising:
a distal cutter assembly (118), comprising:
a housing (202);
a cutting element (204) carried by the housing and comprising at least one cutting blade (300), the cutting element being rotatable relative to the housing to cause the at least one cutting blade to cut the occlusive material and move the occlusive material into the housing;
a catheter (108) coupled to the distal cutter assembly, the catheter comprising:
a sheath (116) configured to receive the occlusive material from the housing;
a drive shaft (216) disposed within the sheath and coupled to the cutting element, the drive shaft being rotatable relative to the sheath to rotate the cutting element relative to the housing, the drive shaft comprising:
an inner coil (510) comprising an inner lumen;
an outer conveyor (518) disposed outwardly from the inner coil, the outer conveyor configured to proximally move the occlusive material in a material removal passageway (218) disposed between the drive shaft and the sheath upon rotation of the drive shaft relative to the sheath; and
a sealing layer (516) disposed between the inner coil and the outer conveyor, the sealing layer configured to inhibit the occlusive material in the material removal passageway from passing through the inner coil and entering the inner lumen.

2. The atherectomy device of claim 1, wherein the sealing layer comprises a polymer.

3. The atherectomy device of claim 2, wherein the polymer is a hydrophobic polymer.

4. The atherectomy device of claim 3, wherein the hydrophobic polymer is one of polytetrafluoroethylene and fluorinate ethylene propylene.

5. The atherectomy device of claim 1, further comprising an intermediate coil (514) disposed between the inner coil and the sealing layer.

6. The atherectomy device of any one of claims 1 to 5, wherein the conveyor is an outer coil and wherein the sealing layer is a jacket.

## Patentansprüche

1. Atherektomievorrichtung (102) zum Entfernen von okklusivem Material von einem Subjekt, wobei die Atherektomievorrichtung umfasst:
eine distale Schneidanordnung (118), umfassend:
ein Gehäuse (202);
ein Schneidelement (204), welches von dem Gehäuse getragen wird und zumindest eine Schneidklinge (300) umfasst, wobei das Schneidelement relativ zu dem Gehäuse drehbar ist, um zu bewirken, dass die zumindest eine Schneidklinge das okklusive Material schneidet und das okklusive Material in das Gehäuse bewegt;
einen Katheter (108), welcher mit der distalen Schneidanordnung gekoppelt ist, wobei der Katheter umfasst:
eine Hülle (116), welche dazu konfiguriert ist, das okklusive Material aus dem Gehäuse aufzunehmen;
eine Antriebswelle (216), welche innerhalb der Hülle angeordnet und mit dem Schneidelement gekoppelt ist, wobei die Antriebswelle relativ zu der Hülle drehbar ist, um das Schneidelement relativ zu dem Gehäuse zu drehen, wobei die Antriebswelle umfasst:
eine innere Spule (510), welche ein inneres Lumen umfasst;
einen äußeren Förderer (518), welcher außerhalb der inneren Spule angeordnet ist, wobei der äußere Förderer dazu konfiguriert ist, das okklusive Material bei Drehung der Antriebswelle relativ zu der Hülle proximal in einem zwischen der Antriebswelle und der Hülle angeordneten Materialentfernungsdurchgang (218) zu bewegen; und
eine Dichtungsschicht (516), welche zwischen der inneren Spule und dem äußeren Förderer angeordnet ist, wobei die Dichtungsschicht dazu konfiguriert ist, das okklusive Material in dem Materialentfernungsdurchgang daran zu hindern, durch die innere Spule zu gelangen und in das innere Lumen einzudringen.

2. Atherektomievorrichtung nach Anspruch 1, wobei die Dichtungsschicht ein Polymer umfasst.

3. Atherektomievorrichtung nach Anspruch 2, wobei das Polymer ein hydrophobes Polymer ist.

4. Atherektomievorrichtung nach Anspruch 3, wobei das hydrophobe Polymer Polytetrafluorethylen oder Fluorethylenpropylen ist.

5. Atherektomievorrichtung nach Anspruch 1, weiter eine Zwischenspule (514) umfassend, welche zwischen der inneren Spule und der Dichtungsschicht angeordnet ist.

6. Atherektomievorrichtung nach einem der Ansprüche 1 bis 5, wobei der Förderer eine äußere Spule ist und wobei die Dichtungsschicht eine Ummantelung ist.

## Revendications

1. Dispositif d'athérectomie (102) pour retirer de la matière occlusive d'un sujet, le dispositif d'athérectomie comprenant :
un ensemble de coupe distal (118), comprenant :
un boîtier (202) ;
un élément de coupe (204) porté par le boîtier et comprenant au moins une lame de coupe (300), l'élément de coupe étant rotatif par rapport au boîtier pour amener la au moins une lame de coupe à couper la matière occlusive et à déplacer la matière occlusive dans le boîtier ;
un cathéter (108) couplé à l'ensemble de coupe distal, le cathéter comprenant :
une gaine (116) configurée pour recevoir la matière occlusive en provenance du boîtier ;
un arbre d'entraînement (216) disposé à l'intérieur de la gaine et couplé à l'élément de coupe, l'arbre d'entraînement pouvant tourner par rapport à la gaine pour faire tourner l'élément de coupe par rapport au boîtier, l'arbre d'entraînement comprenant :
une bobine interne (510) comprenant une lumière interne ;
un transporteur externe (518) disposé à l'extérieur de la bobine interne, le transporteur externe étant configuré pour déplacer de manière proximale la matière occlusive dans un passage d'élimination de matière (218) disposé entre l'arbre d'entraînement et la gaine lors de la rotation de l'arbre d'entraînement par rapport à la gaine ; et
une couche d'étanchéité (516) disposée entre la bobine interne et le transporteur externe, la couche d'étanchéité étant configurée pour empêcher la matière occlusive dans le passage d'élimination de matière de traverser la bobine interne et de pénétrer dans la lumière interne.

2. Dispositif d'athérectomie selon la revendication 1, dans lequel la couche d'étanchéité comprend un polymère.

3. Dispositif d'athérectomie selon la revendication 2, dans lequel le polymère est un polymère hydrophobe.

4. Dispositif d'athérectomie selon la revendication 3, dans lequel le polymère hydrophobe est l'un du polytétrafluoroéthylène et de l'éthylène-propylène fluoré.

5. Dispositif d'athérectomie selon la revendication 1, comprenant en outre une bobine intermédiaire (514) disposée entre la bobine interne et la couche d'étanchéité.

6. Dispositif d'athérectomie selon l'une quelconque des revendications 1 à 5, dans lequel le transporteur est une bobine externe et dans lequel la couche d'étanchéité est une enveloppe.
